Europäisches Patentamt

European Patent Office

Office européen des brevets

(19)

(11) Veröffentlichungsnummer: **0 307 909**

**A1**

(12) **EUROPÄISCHE PATENTANMELDUNG**

(21) Anmeldenummer: 88115120.3

(22) Anmeldetag: 15.09.88

(51) Int. Cl.⁴: **G21C 13/10 , G21C 13/02 ,
G21F 9/04 , C12M 1/12 ,
B01D 50/00 , G21C 15/16 ,
G21C 9/00**

(30) Priorität: 15.09.87 DE 3730955

(43) Veröffentlichungstag der Anmeldung:
22.03.89 Patentblatt 89/12

(84) Benannte Vertragsstaaten:
AT BE CH DE ES FR GB GR IT LI LU NL SE

(71) Anmelder: **Pall Deutschland GmbH
Philipp-Reis-Strasse 6
D-6072 Dreieich 1(DE)**

(72) Erfinder: **Randhahn, Horst, Dr. Ing.
Dresdner Strasse 16
D-6074 Rödermark(DE)**
Erfinder: **Taetz, Frank, Dr. Ing.
Am Dornbusch 35
D-6239 Eppstein(DE)**
Erfinder: **Szymkowiak, Norbert
Dreieichstrasse 25
D-6057 Dietzenbach(DE)**

(74) Vertreter: **Geissler, Bernhard, Dr. jur.,
Dipl.-Phys. Patent- und Rechtsanwälte et al
Bardehle-Pagenberg-Dost-
Altenburg-Frohwitter-Geissler & Partner
Postfach 86 06 20
D-8000 München 86(DE)**

(54) Abscheideeinrichtung für Reaktoren.

(57) Eine Abscheideeinrichtung, insbesondere zur Verwendung in Sicherheitsvorrichtungen von Kernreaktoren oder Bioreaktoren, welche aufweist:

a) einen Druckbehälter, der
   aa) eine Zufuhröffnung
   bb) eine Abfuhröffnung aufweist,
b) einen im Inneren des Druckbehälters angeordneten Tröpfchenabscheider, wobei die abgeschiedene Flüssigkeit aus dem Inneren des Druckgehälters entfernt werden kann,
c) ein im Inneren des Druckbehälters angeordnetes Filter, wobei die Teile so angeordnet sind, daß ein Fluidfluß durch den Druckbehälter von der Zufuhröffnung zu der Abfuhröffnung zuerst durch den Tröpfchenabscheider und danach durch den Filter geht.

Fig. 1

EP 0 307 909 A1

## Abscheideeinrichtung für Reaktoren

Die vorliegende Erfindung bezieht sich auf Abscheideeinrichtungen, nämlich auf Gasreinigungsvorrichtungen, die insbesondere im Sicherheitsbereich von Kernreaktoren oder Bioreaktoren Anwendung finden. Die Erfindung bezieht sich auch auf eine Sicherheitsvorrichtung für derartige bzw. in derartigen Reaktoren.

An Kernreaktoren und Bioreaktoren werden immer höhere Sicherheitsanforderungen gestellt. Bisher wurden mit z.T. riesigem konstruktivem Aufwand Druckbehälter, Ausgleichsbehälter u.ä. konstruiert, um im Falle von Reaktorversagen die Schäden einzudämmen. Im Falle eines katastrophalen Unfalles eines Reaktors, insbesondere also bei einem Schmelzen der spaltbaren Masse eines Kernreaktors, helfen Druckbehälter u.ä. nur begrenzt. Diese können die Kontamination der Atmosphäre nicht verhindern.

Aufgabe der vorliegenden Erfindung ist es, eine Vorrichtung bereitzustellen, mit der Reaktoren versehen werden können, und die im Falle des Betriebes oder von Unfällen in dem Reaktor die Kontamination der Atmosphäre verhindert oder beschränkt.

Diese Aufgabe wird durch eine Abscheideeinrichtung sowie durch eine Sicherheitsvorrichtung gelöst, wie sie in den Ansprüchen definiert sind. Bevorzugte Ausführungsformen sind in den Unteransprüchen definiert.

Die erfindungsgemäße Abscheideeinrichtung weist einen Druckbehälter auf. Dieser Behälter ist so ausgelegt, daß er erheblichen inneren Überdrükken gegenüber der Umgebungsatmosphäre standhält. Insbesondere soll ein solcher Druckbehälter, vorzugsweise beim Einsatz für Druckwasser-Kernreaktoren, dafür ausgelegt sein, einem inneren Überdruck von bis zu 1,6 MPa ohne weiteres Stand zu halten. Für den Einsatz im Zusammenhang mit Bioreaktoren soll ein solcher Druckbehälter vorzugsweise dafür ausgelegt sein, einem inneren Überdruck von bis zu 0,5 MPa ohne weiteres Stand zu halten.

Ein derartiger Druckbehälter besteht vorzugsweise aus rostfreiem Stahl, kann jedoch auch aus Stahlbeton aufgebaut sein. Für die Materialauswahl ausschlaggebend ist deren Gasundurchlässigkeit und deren Hitzebeständigkeit und deren Widerstandsfähigkeit gegen chemische Reaktionen mit Kontaminationsprodukten, insbesondere im Falle von Kernreaktoren also mit Spaltprodukten.

Die Druckbehälter für die Abscheideeinrichtung sind typischerweise im wesentlichen zylindrische Behälter. Typische Abmessungen für die bevorzugten zylindrischen Druckbehälter sind:

- Länge: bis 3 m
- Innendurchmesser: 0,1 bis 2 m
- Wandstärke: 2 bis 20 mm

Die Druckbehälter sind vorzugsweise so ausgebildet, daß sie aus zumindest zwei Teilen aufgebaut sind. Diese beiden Teile sind durch eine Flanschverbindung mit einem Dichtungselement dazwischen miteinander verbindbar.

Im Inneren der erfindungsgemäßen Abscheideeinrichtung befindet sich in Strömungsrichtung gesehen zunächst ein Tröpfchenabscheider. Ein solcher Tröpfchenabscheider soll aus Aerosolen und feuchten Gasströmen, insbesondere bei höheren Temperaturen im Hochdruckbereich die flüssigen Bestandteile abscheiden. Die Tröpfchenabscheider arbeiten überwiegend als Prallabscheider. Die abgeschiedene Flüssigkeit tropft vorzugsweise in den unteren Bereich des Druckbehälters zurück. Von da aus kann die angesammelte Flüssigkeit durch eine Drainageöffnung aus dem Druckbehälter entfernt werden. Von dieser Drainageöffnung fließt die abgeschiedene Flüssigkeit dann durch eine Drainageleitung zu einem Flüssigkeitsaufnahmebehälter.

Der Tröpfchenabscheider kann beispielsweise ein typischer Demister sein. Vorzugsweise weist der Tröpfchenabscheider eine mit dem eintretenden zu reinigenden Strom in Kontakt tretende hydrophobe Oberfläche auf.

In Strömungsrichtung nach dem Tröpfchenabscheider befinden sich in dem Druckbehälter der erfindungsgemäßen Abscheideeinrichtung Filter mit hoher Oberfläche. Für typische Ausführungsformen solcher Abscheideeinrichtungen hat ein solches Filter eine Anströmfläche, also eine Fläche, die das zu reinigende Fluid durchströmt, von ca. 0,2 bis 80 m².

Das bevorzugte Filter für die Abscheideeinrichtung besteht aus einer großen Anzahl zylindrischer Filterkerzen, die in einer Lochplatte im wesentlichen parallel dichtend gehalten werden. Typischerweise befinden sich 1 bis 150 solcher Filterkerzen in einem solchen Druckbehälter. Die Filterkerzen sind hohlzylindrisch aufgebaut, z.B. aus kompakt gefalteten Filtermaterialbahnen, die einen zylindrischen Innenraum umgeben und an dem in den Druckbehälter hineinragenden freien Ende der Filterkerzen durch ein Abschlußstück verschlossen sind. Das untere Ende der Filterkerze ist dichtend in der Lochplatte, z.B. über ein weiteres Abschlußstück der Filterkerze, das eine zentrale Öffnung aufweist, verbunden. Auf diese Weise kann das Fluid von außen durch die Filtermaterialoberfläche einströmen, und das gereinigte Fluid verläßt die Filterkerze durch die zentrale Öffnung in der plattenseitigen Endkappe. Die Filtermaterialien sind vorzugsweise als plissierte Filterelemente ausgebildet und in zylindrischer Form zwischen den Endkappen angeordnet.

Als Filtermaterialien kommen insbesondere Faservliese verschiedener Kombinationen sowie Faservliese kombiniert mit Membranen in Frage. Dabei bestehen die Fasern ebenfalls aus hitzebeständigen und chemikalienbeständigen Materialien. Vorzugsweise werden Vliese aus rostfreiem Stahldraht eingesetzt. Diese Metallfasern können auch mit Kunststoff ummantelt sein. Als Membranen werden vorzugsweise Edelstahlmaterialien oder Fluorkohlenwasserstoffmembranen eingesetzt.

Vorzugsweise besteht das Filtermaterial des Filters aus zumindest zwei Lagen, nämlich einem Metallfasermaterial aus gesintertem Edelstahl und einer daran sich anschließenden Edelstahlmetallmembran.

Vorzugsweise hat das Edelstahlvlies eine absolute Rückhalterate von 3 $\mu$m oder besser und die Edelstahlmembran hat eine absolute Rückhalterate von 5 $\mu$m oder besser, insbesndere von 2 $\mu$m oder besser. Die typischen Edelstahlmembranen, die bei dieser Erfindung vorzugweise eingesetzt werden, haben eine Stärke von 200 bis 500 $\mu$m.

Je nach Anwendungszweck können die Abscheidungseffizienz sowie die Porengröße und das Porenvolumen des Filtermaterials unterschiedlich sein. Bei Abscheidesystemen für Bioreaktoren findet vorzugsweise ein Filtermaterial Anwendung, dessen Abscheideeffizienz für Teilchen von 0,01 $\mu$m und grösser höher ist als $10^9$. Das Filtermaterial zur Anwendung in Bioreaktoren hat vorzugsweise eine absolute Rückhalterate von 0,2 $\mu$m für Flüssigkeiten. Für Filtermaterialien in Abscheidesystemen für Kernreaktoren ist die Abscheideeffizienz $\beta$ für Teilchen von 0,3 $\mu$m und größer vorzugsweise zumindest $10^3$. Im Falle der Filtermaterialien in Abscheidesystemen für Kernreaktoren ist die absolute Rückhalterate vorzugsweise 10 $\mu$m, insbesondere für noch effektivere Abscheidungen etwa 5 $\mu$m und feiner in Flüssigkeiten.

Dabei bedeutet die Abscheideeffizienz $\beta$ das Verhältnis der Anzahl der Partikel (oder Aerosolteilchen) in dem Fluid, das in das Filter eintritt, zu der Anzahl solcher Partikel in dem Fluid, das aus dem Filter austritt. Diese Abscheideeffizienzen gelten je nach Anwendungsbereich für unterschiedliche Zustände wie:
trockene Gase bei Atmosphärendruck
trockene Gase bis 7 bar absolut
feuchte Gase bis 7 bar absolut
für übersättigte Gase.

Insbesondere weisen die beschriebenen Filtermaterialien auch definierte Abscheideeffizienzen bei reiner kontinuierlicher und intermittierender Flüssigkeitsfiltration auf. Die Differenzdruckfestigkeit der Filter bzw. Filterelemente des Abscheidesystems der vorliegenden Erfindung beträgt vorzugsweise bis zu 1 MPa, insbesondere sollte diese Differenzdruckfestigkeit im Bereich von 0,5 bis 1,5 MPa liegen. Diese Angaben gelten insbesondere für die Anwendung der Filter in Kernreaktoren. Bei Bioreaktoren liegen die entsprechenden Werte bei einem Drittel der oben angegebenen Druckwerte.

Für den Einsatz bei Bioreaktoren ist das Filtermaterial vorzugsweise hydrophobes Kunststoffmaterial, insbesondere ein hydrophobes Kunststoffmembranmaterial. Gegenwärtig bevorzugte Materialien bestehen aus fluoriertem Kohlenwasserstoff-Polymeren. Diese Filtermaterialien für Anwendung des Abscheidesystems in Bioreaktoren sind vorzugsweise hydrophob, d.h. ein Tropfen Wasser auf den Materialien darf nicht zerlaufen.

Die bei Bioreaktoren eingesetzten Filtermaterialien sind vorzugsweise Kunststofffilterelemente mit einer Stärke von 100 bis 300 $\mu$m. Zur Erhöhung der Filtereffizienz kann es bevorzugt sein, mit mehrlagigen, insbesondere doppellagigen Filtermaterialien zu arbeiten.

Im Falle eines Abscheidesystems für Bioreaktoren ist es bevorzugt, zwischen dem Tröpfchenabscheider und dem eigentlichen Filter ein Vorfilter zur Entfernung größerer Teilchen einzusetzen. Dieses Vorfilter ist vorzugsweise mit den Abmessungen und Strukturelementen aufgebaut, wie sie oben für das Filtermaterial für die Kernreaktoren angegeben wurden. Dieses Vorfilter kann ebenfalls als Filtereinheit mit einer Vielzahl von hängenden Filterkerzen in einer Lochplatte ausgebildet sein, und das Material für ein derartiges Vorfilter ist vorzugsweise rostfreier Stahl in Form von beispielsweise Edelstahlmembranen bzw. Edelstahlvliesen. Im Betrieb tritt dann der zu reinigende Strom zunächst durch den Tröpfchenabscheider, danach durch das Vorfilter und schließlich durch das eigentliche hydrophobe Filter.

Weitere bevorzugte Einzelheiten und Ausführungsformen der Erfindung ergeben sich aus der folgenden Beschreibung der Zeichnung. Es zeigen
Fig. 1 eine schematische Schnittansicht durch eine Abscheideeinrichtung;
Fig. 2 eine schematische Schnittansicht durch eine Reaktoranlage mit einem Druckwasserreaktor
Fig. 3 und 4 weitere Schnittansichten durch eine Reaktoranlage mit einem Druckwasserreaktor, bei denen die Abscheideeinrichtung gemäß der Erfindung in unterschiedlichen Bereichen, liegt.

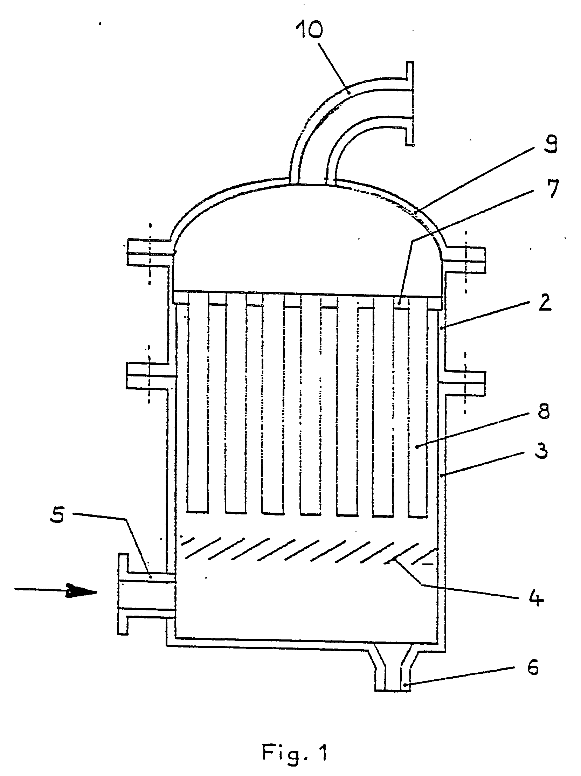

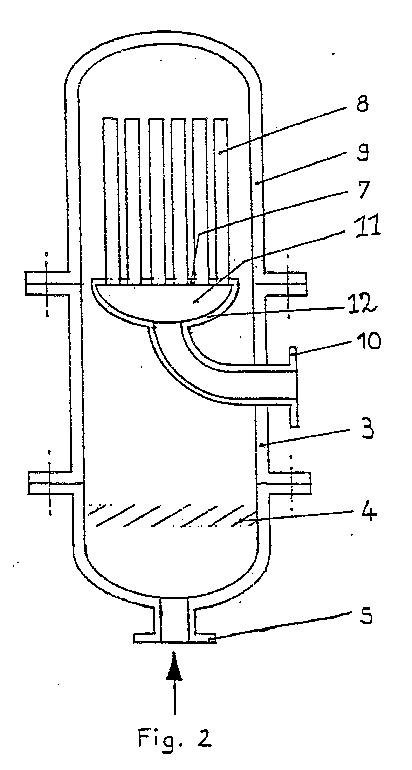

In Fig. 1 ist eine Abscheideeinrichtung gemäß der Erfindung dargestellt. Fig. 2 zeigt die alternative Abscheideeinrichtung mit stehend angeordneten Filterkerzen, die bevorzugt für den Bereich Bioreaktoren ist. Beide weisen einen Druckbehälter 1 auf, der einen oberen Abschnitt 2 und einen unteren Abschnitt 3 aufweist. Im Inneren des Druckbehälters 1 ist in Strömungsrichtung des Fluids gesehen zunächst ein Tröpfchenabscheider 4 angeordnet.

Durch den am Druckbehälter 3 angebrachten Zufuhrstutzen 5 wird das zu reinigende Fluid, in Störfällen meist ein Aerosol mit verschiedenen Verunreinigungen, zugeführt. Es strömt durch den Fluid- oder Tröpfchenabscheider 4. Die sich abscheidende Flüssigkeit tropft zurück in den unteren Bereich des Druckbehälters 1. Vn dort wird die gesammelte Flüssigkeit durch eine Drainagestutzen 6 aus dem Inneren des Druckbehälters 1 abgeführt.

In dem Druckbehälter 1 befindet sich weiterhin eine Platte 7 mit zahlreichen Öffnungen. In diesen Öffnungen der Platte 7 ist jeweils ein im wesentlichen rohrförmiges Filterelement 8 fluiddicht so angebracht, daß das durch den Stutzen 5 eingeführte Fluid, welches durch den Tröpfchenabscheider 4 weitgehend von Tröpfchen befreit wurde, von außen in die Filterkerzen 8 eintritt und das Innere dieser Filterkerzen 8 durch die jeweilige Öffnung in dem Endabschlußstück der Filterkerzen 8 und eine Öffnung in der Lochplatte 7 verläßt. Vorzugsweise sind die oberen Endabschlußstücke der Filterherzen in die Platte 7 eingeschweißt. Der Druckbehälter ist oben durch einen Deckel 9 mit Anschußstutzen 10 verschlossen.

Bei der in Fig. 2 dargestellten und bevorzugt in Bioreaktoren bzw. an Bioreaktoren Anwendung findenden Abscheideeinrichtung sind die Filterkerzen 8 stehend, nicht wie in Fig. 1, hängend angeordnet. Die Platte 7 bildet Teil einer im Inneren des Druckbehälters 3 angeordneten Reinfluid-Sammelkammer 11. Diese Sammelkammer 11 wird im Betrieb von dem vom Tröpfchenabscheider 4 kommenden Fluid umströmt. Das gereinigte Fluid sammelt sich in der Sammelkammer, die insbesondere durch einen schüsselförmigen Abschlußteil 12 zusammen mit der Platte 7 gebildet werden kann. Von dieser Sammelkammer wird das Reinfluid durch eine Reinfluid-Leitung 10, die dichtend durch die Wand des Druckbehälters 3 geführt ist, abgeleitet.

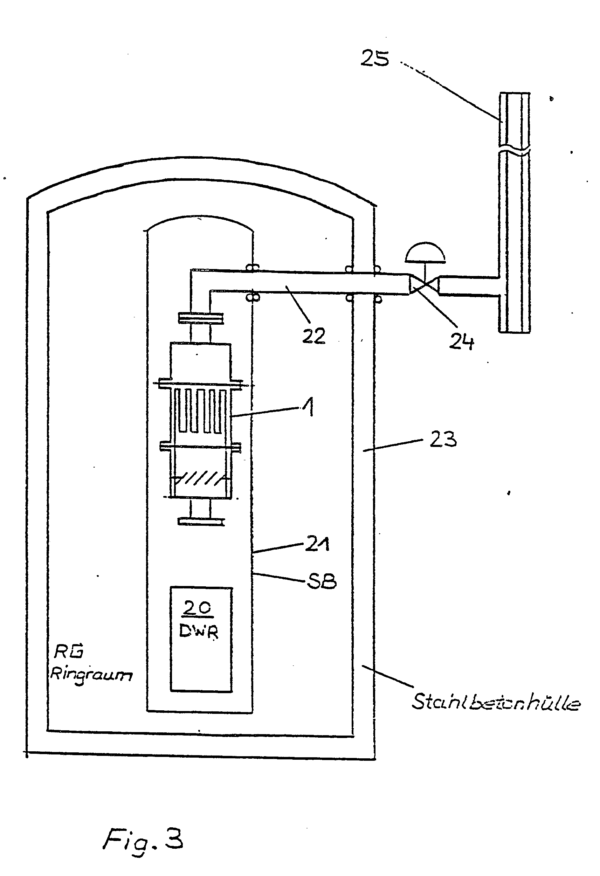

In den Fig. 3, 4 und 5 sind verschiedene Ausführungsformen eines Sicherheitssystems gemäß der vorliegenden Erfindung dargestellt. Dabei ist gemäß Fig. 3 ein Druckwasserreaktor 20 in einem Reaktorraum 21 vorgesehen. Die erfindungsgemäße Abscheideeinrichtung ist innerhalb des Reaktorraumes 21 angeordnet. Eine Abgasleitung 22 führt durch die Wand des Reaktorraumes. Diese Leitung ist dichtend durch diese Wand hindurchgeführt. Die Abgasleitung 22 führt ebenfalls dichtend weiterhin durch die Außenwand des Reaktorringraumes 23 und über ein Druckentspannungsventil 25 zu einer Abgasverarbeitung, z.B. einen Schornstein 25.

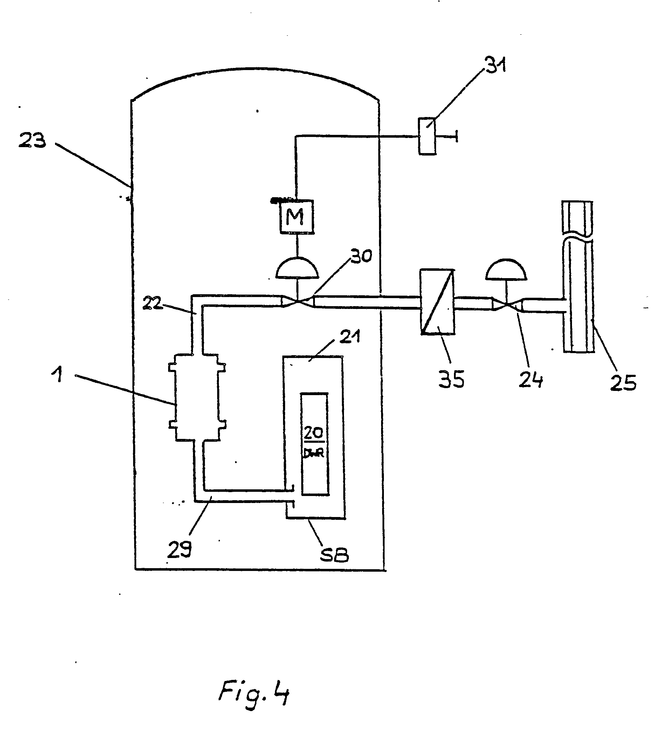

Bei der in Fig. 4 dargestellten Ausführungsform ist ein Druckwasserreaktor 20 wiederum in einem Sicherheitsbehälter 21 vorgesehen. Diesmal ist die Abscheideeinrichtung gemäß der Erfindung innerhalb des Reaktorringraumes 23, aber außerhalb des Reaktorsicherheitsbehälters 21 angeordnet. Die Zufuhrleitung führt in dieser Ausführungsform durch die Wand des Sicherheitsbehälters 21 und zu der Abscheideeinrichtung. Die Abluftleitung 22 führt durch die Wand des Reaktorringraumes 23. In der Abluftleitung 22 ist ein motorbetriebenes Absperrventil 30 vorgesehen. Dieses Motorventil kann über einen Betätigungsschalter 31 von außen betätigt werden. Die Abgasleitung 22 führt bei dieser Ausführungsform noch durch ein weiteres Filtersystem 35 und schließlich durch das Druckentspannungsventil 24 zu dem Kamin 25.

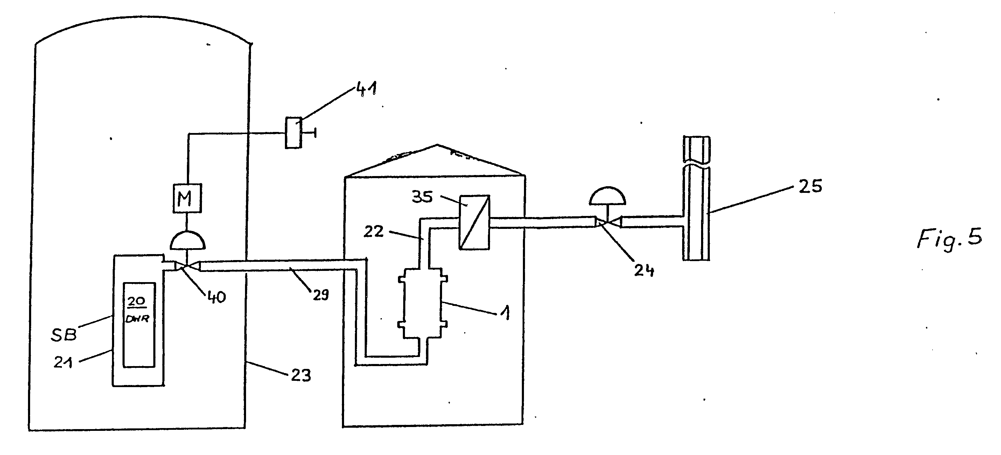

Bei der in Fig. 5 dargestellten Ausführungsform schließlich ist die Abscheideeinrichtung gemäß der Erfindung in einem Bereich außerhalb des Reaktorringraumes 23 angeordnet. Die Zufuhrleitung 29 zu der erfindungsgemäßen Abscheideeinrichtung führt durch die Wand des Sicherheitsbehälters 21, die Wand des Reaktorringraumes 23 und schließlich zu der Abscheideeinrichtung. In dieser Zufuhrleitung 29 ist ein wiederum von außen bedienbares Motorventil 40 vorgesehen, das über einen Schalter 41 betätigt werden kann. Auch bei dieser Ausführungsform ist in der Abgasleitung 22 ein weiteres Filtersystem 35 vorgesehen.

## Ansprüche

1. Abscheideeinrichtung, insbesondere zur Verwendung in Sicherheitsvorrichtungen von Kernreaktoren oder Bioreaktoren, **gekennzeichnet durch**
a) einen Druckbehälter (3), der
aa) eine Zufuhröffnung
bb) eine Abfuhröffnung aufweist,
b) einen im Inneren des Druckbehälters (3) angeordneten Tröpfchenabscheider (4), wobei die abgeschiedene Flüssigkeit aus dem Inneren des Druckbehälters (3) entfernt werden kann,
c) ein im Inneren des Druckbehälters (3) angeordnetes Filter,
wobei die Teile so angeordnet sind, daß ein Fluidfluß durch den Druckbehälter von der Zufuhröffnung zu der Abfuhröffnung zuerst durch den Tröpfchenabscheider (4) und danach durch das Filter geht.

2. Abscheideeinrichtung nach Anspruch 1, dadurch gekennzeichnet, daß der Druckbehälter
cc) eine Drainageöffnung (6) aufweist.

3. Abscheideeinrichtung nach Anspruch 1, dadurch gekennzeichnet, daß der Druckbehälter aus rostfreiem Stahl besteht.

4. Abscheideeinrichtung nach einem der vorhergehenden Ansprüche, dadurch gekennzeichnet, daß der Druckbehälter als im wesentlichen kreiszylindrischer Behälter ausgebildet ist.

5. Abscheideeinrichtung nach Anspruch 4, dadurch gekennzeichnet, daß der Druckbehälter mindestens zwei Teile aufweist, die jeweils einen Ringflansch haben, über die diese zwei Teile miteinander verbunden sind.

6. Abscheideeinrichtung nach einem der vorhergehenden Ansprüche, dadurch gekennzeichnet, daß das Filter aus Fasermaterial besteht.

7. Abscheideeinrichtung nach Anspruch 5, dadurch gekennzeichnet, daß das Fasermaterial Edelstahlfaservlies, vorzugsweise gesintertes Edelstahlfaservlies ist.

8. Abscheideeinrichtung nach einem der vorgehenden Ansprüche, dadurch gekennzeichnet, daß das Filter aus gesintertem Edelstahlfaservlies, angeordnet auf einer gesinterten Edelstahlmembran, besteht.

9. Abscheideeinrichtung nach einem der vorhergehenden Ansprüche, dadurch gekennzeichnet, daß das Filter zumindest eine Filterlage aus hydrophobem Kunststoff, insbesondere eine Fluorkohlenwasserstoffmembran, aufweist.

10. Abscheideeinrichtung nach einem der vorhergehenden Ansprüche, dadurch gekennzeichnet, daß das Filter eine größere Anzahl von parallel geschalteten Filterelementen aufweist.

11. Abscheideeinrichtung nach Anspruch 10, dadurch gekennzeichnet, daß eine Mehrzahl von Filterkerzen (8) in einer quer zur Längsrichtung des Druckbehälters (3) angeordneten Befestigungsplatte (7) befestigt sind.

12. Abscheideeinrichtung nach Anspruch 11, dadurch gekennzeichnet, daß die Filterkerzen (8) je ein kompakt gefältetes Filterelement aufweisen.

13. Abscheideeinrichtung nach einem der vorhergehenden Ansprüche, dadurch gekennzeichnet, daß dem Filter nachgeschaltet ein weiteres Filter vorgesehen ist, das zumindest eine Filterlage aus hydrophobem Kunststoff, insbesondere eine Fluorkohlenwasserstoffmembran, aufweist.

14. Sicherheitsvorrichtung für einen Reaktor, dadurch gekennzeichnet,

a) daß eine Abscheideeinrichtung nach einem der vorhergehenden Ansprüche vorgesehen ist,

b) daß die Zufuhröffnung der Abscheideeinrichtung mit dem Inneren einer Sicherheitszone des Reaktors in Strömungsverbindung steht,

c) daß die Abflußöffnung der Abscheideeinrichtung mit dem Eingang einer Druckreduziereinrichtung verbunden ist, und

d) daß der Ausgang der Druckreduziereinrichtung mit einer Abgasbehandlungseinrichtung in Strömungsverbindung steht.

15. Sicherheitsvorrichtung nach Anspruch 14, dadurch gekennzeichnet, daß die Abscheideeinrichtung im Inneren des innersten Sicherheitsbehälters des Reaktors angeordnet ist.

16. Sicherheitsvorrichtung nach Anspruch 14, dadurch gekennzeichnet, daß die Abscheideeinrichtung in dem den innersten Sicherheitsbehälter umgebenden Reaktorraum angeordnet ist, daß eine Fluid zuleitende Rohrleitung von der Zufuhröffnung der Abscheideeinrichtung in den innersten Sicherheitsbehälter führt, und daß eine Gas ableitende Rohrverbindung durch die Wand des Reaktorraumes führt.

17. Sicherheitsvorrichtung nach Anspruch 14, dadurch gekennzeichnet, daß die Abscheideeinrichtung außerhalb des Reaktorraumes angeordnet ist, daß eine Fluid zuleitende Rohrverbindung zwischen dem Inneren des innersten Sicherheitsbehälters des Reaktors und der Zuführöffnung der Abscheideeinrichtung besteht, die durch die Wand des innersten Sicherheitsbehälters und durch die Wand des Reaktorraumes dichtend geführt ist.

18. Sicherheitsvorrichtung nach Anspruch 16 oder 17, dadurch gekennzeichnet, daß in der Fluid zuführenden Rohrverbindung zumindest ein von außerhalb betätigbares Motorventil (Regelventil) in dem Abschnitt der Rohrverbindung angeordnet ist, die sich im Reaktorraum befindet.

19. Sicherheitsvorrichtung nach einem der Ansprüche 13 bis 16, dadurch gekennzeichnet, daß in der Rohrverbindung, die das Gas ableitet, zumindest eine weitere Filterstrecke vorgesehen ist.

20. Sicherheitsvorrichtung nach Anspruch 17, dadurch gekennzeichnet, daß die weitere Filterstrecke ausgewählt ist aus der Gruppe bestehend aus

a) Adsorptionsfilter

b) hocheffizienter Partikelfiltern und

c) mikrobiologischen Sicherheitsfiltern.

21. Sicherheitsvorrichtung nach Anspruch 18, dadurch gekennzeichnet, daß eine allgemeine Notfall-Filtereinrichtung mit der Gas ableitenden Rohrverbindung verbunden ist, derart, daß das austretende gefilterte Gas zumindest teilweise durch diese Notfall-Filtereinrichtung geleitet wird.

22. Verwendung einer Sicherheitsvorrichtung nach einem der Ansprüche 13 bis 20 bei einem Kernreaktor, insbesondere einem Druckwasserreaktor.

23. Verwendung einer Sicherheitsvorrichtung nach einem der Ansprüche 13 bis 20 bei einem Bioreaktor.

24. Abscheideeinrichtung nach Anspruch 11 oder 12, dadurch gekennzeichnet, daß

a) die Filterkerzen (8) an der Befestigungsplatte (7) von dem Tröpfchenabscheider (4) wegweisend angeordnet sind,

b) die Befestigungsplatte (7) Teil einer Reinfluid-Sammelkammer (11) ist, die im Inneren des Druckbehälters (3) zwischen den Filterkerzen (8) und dem Tröpfchenabscheider (4) angeordnet ist, und von dem zu reinigenden Fluid, das von dem Tröpfchenabscheider (4) kommt, umströmt wird,

c) die Reinfluid-Sammelkammer mit einer Reinfluid-Leitung (10) verbunden ist, die dichtend durch die Wand des Druckbehälters (3) geführt ist.

Fig. 1

Fig. 2

25

22

24

1

23

21

SB

20
DWR

RG
Ringraum

Stahlbetonhülle

Fig.3

Fig. 4

Fig.5

EP 0 307 909 A1

## EINSCHLÄGIGE DOKUMENTE

EP 88115120.3

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| X | DE - A - 1 503 418 (P. GRAEFE)<br> * Ansprüche; Fig. * <br> -- | 1,2,4, 5 | G 21 C 13/10<br>G 21 C 13/02<br>G 21 F 9/04 |
| X | US - A - 4 478 619 (ARENDS et al.)<br> * Ansprüche * <br> -- | 1,14 | C 12 M 1/12<br>B 01 D 50/00<br>G 21 C 15/16 |
| A | CH - A - 483 864 (BROWN BOVERIE)<br> * Gesamt * <br> -- | 1,14 | G 21 C 9/00 |
| A | DE - B - 1 082 992 (COMMISSARIAT A L'ENERGIE ATOMIQUE)<br> * Gesamt * <br> -- | 1,14 | |
| A | DE - B - 1 274 254 (LICENTIA)<br> * Gesamt * <br> -- | 1,14 | |
| A | DE - A - 2 104 356 (SIEMENS)<br> * Gesamt * <br> -- | 1,14 | RECHERCHIERTE SACHGEBIETE (Int. Cl.4)<br><br>G 21 C 9/00<br>G 21 C 13/00<br>G 21 C 15/00 |
| A | DE - A - 2 203 107 (INTERATOM)<br> * Gesamt * <br> -- | 1,14 | G 21 F 9/00<br>C 12 M 1/00<br>B 01 D 40/00<br>B 01 D 50/00 |
| A | DE - A1 - 3 247 580 (NIPPIES »ENERGOPROEKT«)<br> * Gesamt * <br> -- | 1,14 | |

Der vorliegende Recherchenbericht wurde für alle Patentansprüche erstellt.

| Recherchenort<br>WIEN | Abschlußdatum der Recherche<br>17-11-1988 | Prüfer<br>BAUMANN |
|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| | **EINSCHLÄGIGE DOKUMENTE** | | EP 88115120.3 |
| A | GB - A - 1 458 390 (PENNWALT) <br> * Ansprüche * <br><br> -- | 1,14 | |
| A | EP - A2 - 0 007 133 (CHEMAP) <br> * Zusammenfassung * <br><br> -- | 1,4,10 | |
| A | EP - A2 - 0 135 208 (H. MÜLLER AG) <br> * Zusammenfassung * <br><br> ---- | 1,4,10 | |

RECHERCHIERTE
SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| WIEN | 17-11-1988 | BAUMANN |